# EUROPEAN PATENT APPLICATION

(11) **EP 3 239 291 A1**
(43) Date of publication of application: **01.11.2017**
(21) Application number: 16000936.1
(22) Date of filing: 26.04.2016
(51) Int. Cl.: C12N 1/20

(54) **BACTERIAL STRAIN AND METHOD FOR HIGH THROUGHPUT OF SUGAR IN THE MICROBIAL CONVERSION INTO BIOSYNTHETIC PRODUCTS**

(71) Applicant: Universität Stuttgart, 70174 Stuttgart (DE)
(72) Inventor: Michalowski, Annette, 49074 Osnabrück (DE); Siemann-Herzberg, Martin, 75365 Calw (DE); Takors, Ralf, 70569 Stuttgart (DE)
(74) Representative: Müller-Boré & Partner Patentanwälte PartG mbB

(57) **Abstract**

The present invention relates to recombinant *Escherichia coli* (*E*. *coli*) host cells comprising, in relation to wild-type cells, at least one mutation selected from the group consisting of deletion of the gene *relA* (Δ*relA*); amino acid substitutions R290E and K292D in the protein guanosine-3',5'-bis pyrophosphate 3'-pyrophosphohydrolase (bifunctional (p)ppGpp synthetase II; SpoT) (spo*T*[R290E;K292D]); and amino acid substitution G267C in the protein pyruvate dehydrogenase subunit E1 (AceE) (ace*E*[G267C]). Said recombinant host cells are characterized by increased sugar uptake rates that lead to increased productivity when using said cells for the production of biosynthetic products. The present invention further relates to respective methods for the biosynthetic production of a product of interest using said host cells.

## Description

The present invention relates to recombinant *Escherichia coli (E. coli)* host cells comprising, in relation to wild-type cells, at least one mutation selected from the group consisting of deletion of the gene *relA* (Δ*relA*); amino acid substitutions R290E and K292D in the protein guanosine-3',5'-bis pyrophosphate 3'-pyrophosphohydrolase (bifunctional (p)ppGpp synthetase II; SpoT) (*spoT*[R290E;K292D]); and amino acid substitution G267C in the protein pyruvate dehydrogenase subunit E1 (AceE) (*aceE*[G267C]). Said recombinant host cells are characterized by increased sugar uptake rates that lead to increased productivity when using said cells for the production of biosynthetic products. The present invention further relates to respective methods for the biosynthetic production of a product of interest using said host cells.

Aerobic industrial production processes in microbial systems generally are subject to technical limitations concerning oxygen transfer rates and efficient cooling of the process. For this reason, such processes usually have to be carried out under conditions of reduced metabolic activity of the cells during the actual production phase. In this manner, the production process can be maintained within the boundaries of technical limitations. However, productivity of the process, *i.e.,* the amount of generated product per reactor volume and time, is also reduced.

The reduction of the metabolic activity of cells is conventionally achieved by specifically adjusted substrate limitations, e.g. of sugar, nitrogen sources, or phosphate, or by the use of suboptimal temperatures or pH values. For these reasons, the production phase is usually characterized by a strong limitation of cell growth.

However, in addition to a limitation of cell growth, the reduction of metabolic activity usually also leads to a reduction of glucose uptake rates of the cells. Therefore, concurrently with a reduction of absolute sugar uptake, a larger percentage of sugar metabolism is needed for the maintenance of essential cell functions. Accordingly, not only process productivity is inherently reduced, but in many cases also product yield.

Thus, the need for significantly increased sugar uptake rates at low growth rates arises. This would increase sugar turnover in microbial producers, so that ingested sugar could be directed intracellularly to biosynthetic routes of interest. This could lead to a significant increase in process productivity compared to conventional processes. Thus, significantly increased metabolic rates could be realized while at the same time observing the same technical limitations discussed above. This could lead to a novel microbial production platform that could be applicable to all kinds of processes for the microbial production of a wide range of products of interest.

Therefore, the technical problem underlying the present invention is the provision of recombinant microbial host cells displaying significantly increased sugar uptake rates at low growth rates, as well as production processes using the same.

The solution to the above technical problem is achieved by the embodiments characterized in the claims.

In particular, in a first aspect, the present invention relates to a recombinant *Escherichia coli* (*E. coli*) host cell, wherein said cell comprises at least one of the following mutations in relation to a wild-type cell:
(i) deletion of the gene *relA* (Δ*relA*);
(ii) amino acid substitutions R290E and K292D in the protein guanosine-3',5'-bis pyrophosphate 3'-pyrophosphohydrolase (bifunctional (p)ppGpp synthetase II; SpoT) (*spoT*[R290E;K292D]); and
(iii) amino acid substitution G267C in the protein pyruvate dehydrogenase subunit E1 (AceE) (*aceE*[G267C]).

As used herein, the term "recombinant cell" refers to a cell whose genome has been artificially altered as compared to a wild-type cell.

*E. coli* strains that are encompassed in the present invention are not particularly limited and respective strains are known in the art. In preferred embodiments, the recombinant host cell is derived from *E. coli* strain *E. coli* K-12, more preferably from *E. coli* strain *E. coli* K-12 substrain MG1655.

The term "mutation" as used herein relates to any permanent alteration of the nucleotide sequence of the genome of the host cell. Further, the term "mutation in relation to a wild-type cell" as used herein relates to the fact that said mutation is not present in a wild-type cell, but is present in the host cells of the present invention.

The first mutation that can be present in the host cells of the present invention is deletion of the gene *relA* (Δ*relA*). The term "deletion" as used herein relates to the removal of any number of nucleotides that leads to a complete abolishment of the expression of functionally active protein. By way of example, deletion of a gene may encompass removal of the entire gene or the entire coding sequence, or removal of only few or a single nucleotide(s) leading to a complete abolishment of expression or a total loss of protein activity.

Said gene *relA* encodes the enzyme (p)ppGpp synthetase I (also known as GTP pyrophosphokinase). Sequence information for this gene can be found under EcoGene Accession Number EG10835. Sequence information for the respective protein can be found under UniProtKB/Swiss-Prot Accession Number P0AG20. The enzyme (p)ppGpp synthetase I catalyzes the conversion of ATP and GTP into pppGpp by adding the pyrophosphate from ATP onto the 3' carbon of the ribose in GTP releasing AMP. Thus, said enzyme is a key mediator of the *E*. *coli* stringent response, which is a stress response in reaction to amino-acid starvation, fatty acid limitation, iron limitation, heat shock and other stress conditions. The stringent response is signaled by the alarmone (p)ppGpp (guanosine penta- or tetraphosphate), and modulates transcription of up to 1/3 of all genes in the cell. This in turn causes the cell to divert resources away from growth and division and toward amino acid synthesis in order to promote survival until nutrient conditions improve.

Methods for gene deletion in *E. coli* are not particularly limited and are known in the art.

The second mutation that can be present in the host cells of the present invention is the presence of amino acid substitutions R290E and K292D in the protein guanosine-3',5'-bis pyrophosphate 3'-pyrophosphohydrolase (bifunctional (p)ppGpp synthetase II; SpoT) (*spoT*[R290E;K292D]). Said protein is encoded by the gene *spoT.* Sequence information for this gene can be found under EcoGene Accession Number EG10966. Sequence information for the respective protein can be found under UniProtKB/Swiss-Prot Accession Number P0AG24. The bifunctional enzyme (p)ppGpp synthetase II catalyzes the hydrolysis as well as the synthesis of (p)ppGpp. Thus, said enzyme is an important regulator of the *E. coli* stringent response.

Methods for introducing amino acid substitutions in a particular protein are not particularly limited and are known in the art. They include any methods of altering the respective coding sequence so that the substitute amino acid instead of the wild-type amino acid is encoded.

The third mutation that can be present in the host cells of the present invention is the presence of amino acid substitution G267C in the protein pyruvate dehydrogenase subunit E1 (AceE) (*aceE*[G267C]). Said protein is encoded by the gene *aceE.* Sequence information for this gene can be found under EcoGene Accession Number EG10024. Sequence information for the respective protein can be found under UniProtKB/Swiss-Prot Accession Number P0AFG8. Pyruvate dehydrogenase subunit E1 is a part of the *E. coli* pyruvate dehydrogenase complex (PDC) which is a complex of three enzymes that convert pyruvate into acetyl-CoA by pyruvate decarboxylation. Acetyl-CoA may then be used in the citric acid cycle to carry out cellular respiration, so that this complex links the glycolysis metabolic pathway to the citric acid cycle.

In preferred embodiments, the host cells of the present invention comprise the above mutation (i). In other preferred embodiments, said host cells comprise at least two of the above mutations (i) to (iii), preferably mutations (i) and (ii). In particularly preferred embodiments, said host cells comprise all three of the above mutations (i) to (iii).

In a second aspect, the present invention relates to a method for the biosynthetic production of a product of interest (POI), wherein said POI is produced in a recombinant host cell according to the present invention.

The term "biosynthetic production" as used herein relates to the fact that the POI is produced via endogenous biosynthesis in the host cells of the present invention or by help of said host cells.

The POI does not underlie any specific restrictions, provided it can be biosynthetically produced in the host cells of the present inventions. In particular embodiments, the POI is a protein and said protein is expressed in the host cells of the present invention. In other particular embodiments, the POI is an organic molecule and said organic molecule is produced in the host cells of the present invention as a metabolite. In preferred embodiments, the organic molecule is selected from the group consisting of pyruvate, lactate, and acetate. In other preferred embodiments, the organic molecule is a molecule that benefits from a high precursor supply from the central metabolism, as well as from higher energy supply in the form of ATP and reduction equivalents such as NADH/NADPH, due to elevated catabolic activities in the host cell.

As used herein the term "produced in the host cells as a metabolite" relates to the fact that the POI is the result of a particular metabolic pathway of the host cells of the present invention. This metabolic pathway may be an endogenous pathway that is already present in wild-type cells, or an engineered pathway that is implemented or modified transgenically. In preferred embodiments, the starting material for the production of said metabolite is a sugar, preferably a sugar selected from the group consisting of glucose, fructose, mannose, xylose, arabinose, and sucrose, wherein glucose is particularly preferred. In other preferred embodiments, the starting material is a substrate that is metabolized in the central metabolism of *E. coli,* preferably a substrate that is transported into the cell via the phosphotransferase system (PTS).

In preferred embodiments, the method of the present invention is be carried out under conditions of reduced metabolic activity of the cells during the actual production phase, in order to maintain the production process within the boundaries of the technical limitations described above. Preferably, metabolic activity is reduced by limiting the amount of available nitrogen sources.

Preferably, the methods of the present invention as defined above comprise the steps of:
(a) providing a host cell of the present invention, wherein said host cell is capable of producing the POI,
(b) culturing said host cell under conditions allowing production of the POI.

Means of rendering a host cell capable of producing one or more particular POI do not underlie any specific restrictions and are known in the art. Further, respective culture techniques and conditions are known in the art. By way of example, such techniques include batch or continuous flow processes in bioreactors, fed-batch processes with our without cell retention, immobilized or submerged cultivated cells, and *E. coli* biofilms, optionally in an industrial scale.

In a third aspect, the present invention relates to the use of a recombinant host cell of the present invention for the biosynthetic production of a product of interest (POI).

In this aspect, all relevant definitions and limitations defined above for the host cells and the methods of the present invention apply in an analogous manner.

The term "comprise(s)/comprising" as used herein is expressly intended to encompass the terms "consist(s)/consisting essentially of" and "consist(s)/consisting of".

By specific targeted interventions into *E. coli* metabolism and metabolic regulation, the present invention advantageously achieves a two- to three-fold increase of sugar uptake rates in resting, non-growing cells as compared to wild-type cells. This results in a two- to three-fold increased carbon source flow in the cells which can supply processes for the production of a microbial product of interest. Thus, process productivity can be increased by the same factor.

The figures show:

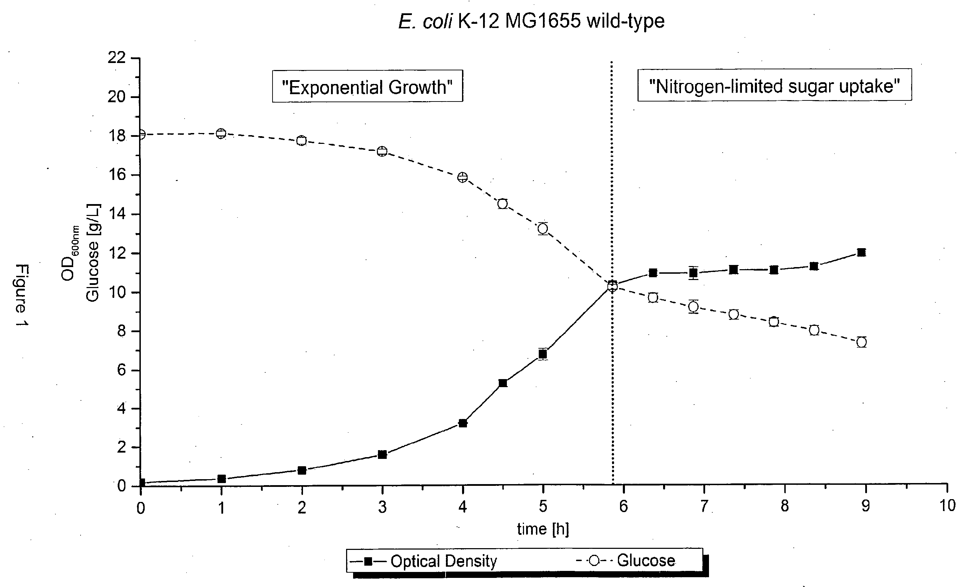

Figure 1: Batch cultivation of the *Escherichia coli* MG1655 wild-type strain in a minimal medium supplemented with 18 g/L glucose as sole C-source and 40 mM NH₄⁺ as sole N-source at starting conditions. After 6 hours glucose is still in excess and the nitrogen source is consumed to a minimum residual concentration. Exponential bacterial growth stops immediately. Data points and error bars derive from three parallel fermentations n=3.

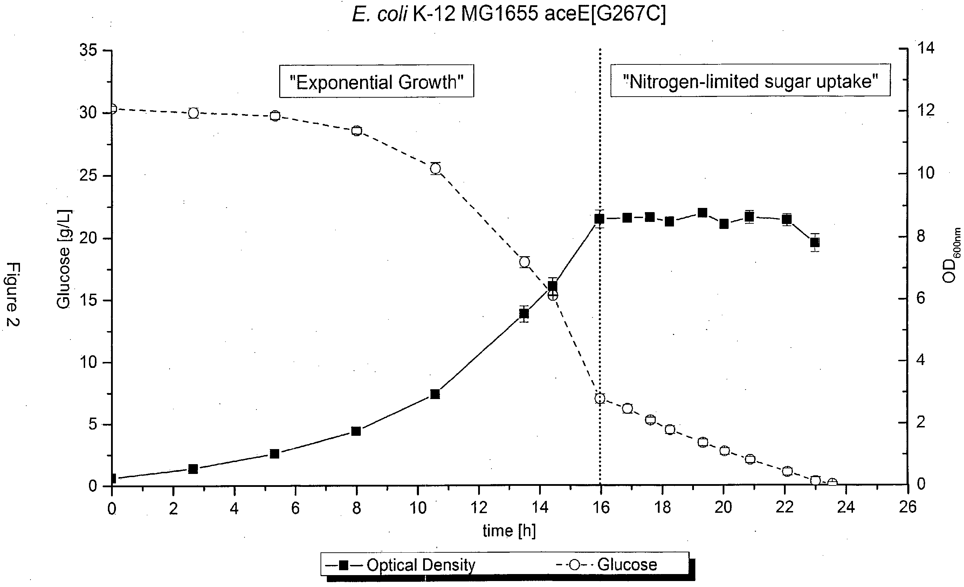

Figure 2: Batch cultivation of the *Escherichia coli* K-12 MG1655 *aceE*[G267C] strain in a minimal medium supplemented with 30 g/L glucose as sole C-source and 40 mM NH₄⁺ as sole N-source at starting conditions. After 16 hours glucose is still in excess and the nitrogen source is consumed to a minimum residual concentration. Exponential bacterial growth stops immediately. Data points and error bars derive from three parallel fermentations n=3.

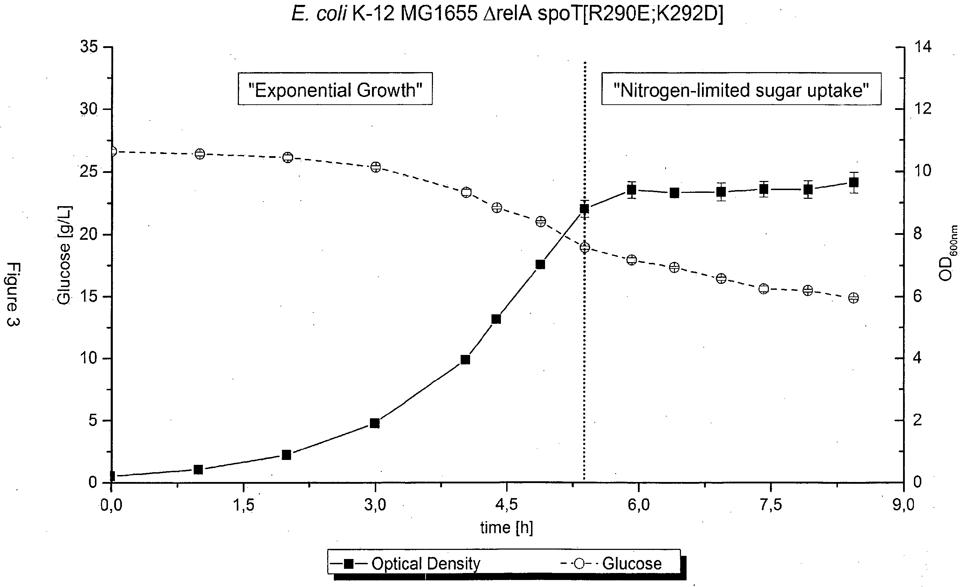

Figure 3: Batch cultivation of the *Escherichia coli* K-12 MG1655 Δ*relA spoT*[R290E;K292D] strain in a minimal medium supplemented with 28 g/L glucose as sole C-source and 40 mM NH₄⁺ as sole N-source at starting conditions. After 5.4 hours glucose is still in excess and the nitrogen source is consumed to a minimum residual concentration. Exponential bacterial growth stops immediately. Data points and error bars derive from three parallel fermentations n=3.

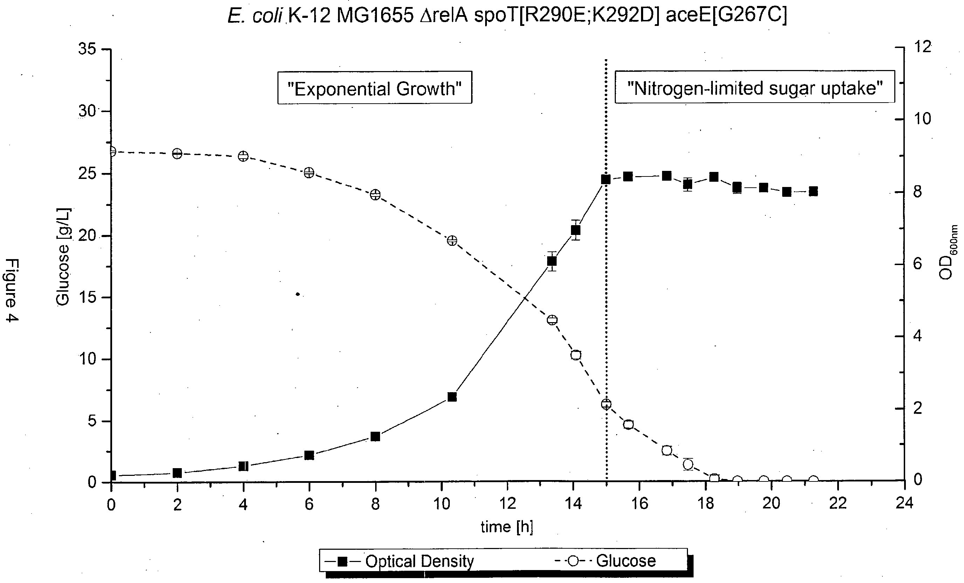

Figure 4: Batch cultivation of the *Escherichia coli* K-12 MG1655 Δ*relA spoT*[R290E;K292D] *aceE*[G267C] strain in a minimal medium supplemented with 28 g/L glucose as sole C-source and 40 mM NH₄⁺ as sole N-source at starting conditions. After 15 hours glucose is still in excess and the nitrogen source is consumed to a minimum residual concentration. Exponential bacterial growth stops immediately. Data points and error bars derive from three parallel fermentations n=3.

The present invention will be further illustrated by the following examples without being limited thereto.

### Examples

### Material and Methods:

### Media and solutions - Preculture minimal medium

**Solution A: (10x Salts)**

| | |
|---|---|
| NaH₂PO₄ · 2 H₂O | 98.44 g/L |
| K₂HPO₄ | 46.86 g/L |
| (NH₄)₂HPO₄ | 13.21 g/L |
| (NH₄)₂SO₄ | 26.80 g/L |
| Na₂SO₄ | 8.80 g/L |
| pH was adjusted to pH 7.0 with KOH | |

**Solution B: (1000x Ca²⁺)**

| | |
|---|---|
| CaCl₂ · 2 H₂O | 14.70 g/L |

**Solution C: (1000x Mg²⁺)**

| | |
|---|---|
| MgSO₄ · 7 H₂O | 246.48 g/L |

**Solution D: (2000x Trace Elements Solution = TES)**

| | |
|---|---|
| FeCl₃ · 6 H₂O | 16.70 g/L |
| Na₂-EDTA | 20.10 g/L |
| ZnSO₄ · 7 H₂O | 0.18 g/L |
| MnSO₄ · H₂O | 0.10 g/L |
| CuSO₄ · 5 H₂O | 0.16 g/L |
| CoCl₂ · 6 H₂O | 0.18 g/L |

**Solution E: (1000x Vitamin)**

| | |
|---|---|
| Thiamine HCl | 10.00 g/L |

**Solution F: (50% glucose w/v)**

| | |
|---|---|
| α-D(+)-Glucose · H₂O | 500.00 g/L |

### Media and solutions - Batch minimal medium

**Solution A.2: (10x Salts)**

| | |
|---|---|
| NaH₂PO₄ · 2 H₂O | 98.44 g/L |
| K₂HPO₄ | 46.86 g/L |
| (NH₄)₂HPO₄ | 13.21 g/L |
| (NH₄)₂SO₄ | 12.68 g/L |
| Na₂SO₄ | 8.80 g/L |
| pH was adjusted to pH 7.0 with KOH | |

**Solution B: (1000x Ca²⁺)**

| | |
|---|---|
| CaCl₂ · 2 H₂O | 14.70 g/L |

**Solution C: (1000x Mg²⁺)**

| | |
|---|---|
| MgSO₄ · 7 H₂O | 246.48 g/L |

**Solution D: (2000x Trace Elements Solution = TES)**

| | |
|---|---|
| FeCl₃ · 6 H₂O | 16.70 g/L |
| Na₂-EDTA | 20.10 g/L |
| ZnSO₄ · 7 H₂O | 0.18 g/L |
| MnSO₄ · H₂O | 0.10 g/L |
| CuSO₄ · 5 H₂O | 0.16 g/L |
| CoCl₂ · 6 H₂O | 0.18 g/L |

**Solution E: (1000x Vitamin)**

| | |
|---|---|
| Thiamine HCl | 10.00 g/L |

**Solution F: (50% glucose w/v)**

| | |
|---|---|
| α-D(+)-Glucose · H₂O | 500.00 g/L |

### Preculture shaking flask cultivation

To prepare the preculture minimal medium, solutions A, B and C, described above, were prepared separately and also separately sterilized at 120°C for 20 min. Solutions D, E and F were separately prepared and sterile filtrated at 0.2 µm pore size. For 1 L of ready-to-use preculture medium 100 mL 10x salts, 1 mL 1000x Ca²⁺ stock solution, 1 mL 1000x Mg²⁺ stock solution, 0.5 mL 2000x TES, 1 mL 1000x Vitamin stock solution, 10 mL 50% w/v glucose stock solution and 886.5 mL sterile water were mixed. Sterile 500 mL Erlenmeyer shaking flasks with baffles were filled with 60 mL of the preculture minimal medium. For each strain the preculture was carried out in parallel with three uniquely inoculated shaking flasks at 37°C and constant agitation. After incubation, the bacterial cells were harvested by centrifugation (4500 x g, 10 min, 4°C) and diluted to an Optical Density of about 8.0 in a volume of 5 mL. This cell suspension was used for inoculation of the bioreactors.

### Batch cultivation

To prepare the batch minimal medium, solutions B and C, described above, were prepared separately and also separately sterilized at 120°C for 20 min. Solutions D, E and F were separately prepared and sterile filtrated at 0.2 µm pore size. All fermentation processes were carried out in a parallel cultivation system consisting of three identical HWS glass bioreactors with a working volume of 250 mL each. After assemblage of the cultivation system, every bioreactor was separately filled with 20 mL of 10x salts (solution A.2) and 160 mL of water. This diluted salt solution was sterilized in every bioreactor at 120°C for 20 min. After sterilization a total volume of 15 mL containing: 7.2 mL 50% w/v glucose stock solution (*E*. *coli* K-12 MG1655 wild-type) or 11.2 mL 50% w/v glucose stock solution (*E. coli* K-12 MG1655 Δ*relA spoT*[R290E;K292D], *E*. *coli* K-12 MG1655 Δ*relA spoT*[R290E;K292D] *aceE*[G267C]) or 12 mL 50% w/v glucose stock solution (*E*. *coli* K-12 MG1655 *aceE*[G267C]), 0.2 mL 1000x Ca²⁺ stock solution, 0.2 mL 1000x Mg²⁺ stock solution, 0.1 mL 2000x TES, 0.2 mL 1000x Vitamin stock solution and 7.1 mL water or 3.1 mL water or 2.3 mL water, respectively, was added sterile to every vessel. Each bioreactor was inoculated with 5 mL of a concentrated preculture giving a starting Optical Density of 0.2. Fermentations were performed at a constant temperature of 37°C, agitation and good oxygen supply. The process length varied for every *E*. *coli* K-12 MG1655 strain. Individual fermentation durations are mentioned for the corresponding strains in Examples 1 to 4, below.

### Nitrogen-limited batch cultivation phase

Each and every batch cultivation process started with identical conditions, except of varying initial glucose concentrations for the processes of *E*. *coli* K-12 MG1655 wild-type/*E*. *coli* K-12 MG1655 Δ*relA spoT*[R290E;K292D]) and *E*. *coli* K-12 MG1655 *aceE*[G267C]/*E*. *coli* K-12 MG1655 Δ*relA spoT*[R290E;K292D] *aceE*[G267C]. Despite the actual amount of glucose, this sole C-source was always in vast excess at the beginning of every fermentation. This also extends to all additional nutrients in the batch minimal medium, as listed above. For the first couple of hours every *E. coli* K-12 MG1655 strain was growing exponentially at its very specific maximum growth rate and consumed glucose with its individual biomass-specific uptake rate under non-limited conditions. This state is termed as "Exponential Growth" in Figures 1, 2, 3 and 4. A fixed nitrogen concentration in the minimal medium enables the bacterial cells to form a certain total biomass before entering nitrogen-depleted nutritional conditions. The subsequent N-limited growth phase is further termed as "Nitrogen-limited Growth" in Figures 1, 2, 3 and 4. During this late stage of the fermentation process bacterial growth was highly limited due to nitrogen-depletion. However, the glucose concentration remained abundantly and the rates for biomass-specific glucose consumption under limited growth conditions could be calculated.

### Example 1:

In this example *Escherichia coli* K-12 MG1655 wild-type was cultivated under preculture conditions in shaking flasks, as described above, for 12 hours with constant agitation. These bacterial cells were then transferred into the three bioreactors under sterile conditions to start the batch cultivation process. *Escherichia coli* K-12 MG1655 wild-type cells were cultivated for a total period of 9 hours with a starting concentration of glucose being 18 g/L. In the exponential growth phase the maximal biomass-specific growth rate was 0.718 ± 0.007 h⁻¹ and glucose was consumed at a biomass-specific rate of 1.765 ± 0.056 g_{Glc}/g_{cdw}·h (cdw: cell dry weight). As can be seen in Figure 1, these cells were growing exponentially during the first 6 hours of cultivation before all the NH₄⁺ in the minimal medium was depleted and the nitrogen-limited growth phase was reached. During the following 3 hours of cultivation the bacterial cells showed a limited linear growth behavior and also a linear progression of glucose consumption. The biomass-specific glucose uptake rate in the nitrogen-limited cultivation phase from hour 6 to 9 averaged at a value of 0.245 ± 0.011 g_{Glc}/g_{cdw}·h and the biomass-specific growth rate dropped to a value of 0.043 ± 0.004 h⁻¹.

### Example 2:

In this example *Escherichia coli* K-12 MG1655 *aceE*[G267C] was cultivated under preculture conditions in shaking flasks, as described above, for 29.5 hours with constant agitation. These bacterial cells were then transferred into the three bioreactors under sterile conditions to start the batch cultivation process. *Escherichia coli* K-12 MG1655 *aceE*[G267C] cells were cultivated for a total period of 23.5 hours with a starting concentration of glucose being 30 g/L. In the exponential growth phase the maximal biomass-specific growth rate was 0.201 ± 0.004 h⁻¹ and glucose was consumed at a biomass-specific rate of 1.512 ± 0.022 g_{Glc}/g_{cdw}·h. As can be seen in Figure 2, these cells were growing exponentially during the first 16 hours of cultivation before all the NH₄⁺ in the minimal medium was depleted and the nitrogen-limited growth phase was reached. During the following 7.5 hours of cultivation the bacterial cells showed a limited linear growth behavior and also a linear progression of glucose consumption. The biomass-specific glucose uptake rate in the nitrogen-limited cultivation phase from hour 16 to 23.5 averaged at a value of 0.314 ± 0.012 g_{Glc}/g_{cdw}·h and the biomass-specific growth rate dropped to a value of 0.008 ± 0.004 h⁻¹.

### Example 3:

In this example *Escherichia coli* K-12 MG1655 Δ*relA spoT*[R290E;K292D] was cultivated under preculture conditions in shaking flasks, as described above, for 11 hours with constant agitation. These bacterial cells were then transferred into the three bioreactors under sterile conditions to start the batch cultivation process. *Escherichia coli* K-12 MG1655 Δ*relA spoT*[R290E;K292D] cells were cultivated for a total period of 8.4 hours with a starting concentration of glucose being 28 g/L. In the exponential growth phase the maximal biomass-specific growth rate was 0.715 ± 0.003 h⁻¹ and glucose was consumed at a biomass-specific rate of 1.770 ± 0.059 g_{Glc}/g_{cdw}·h. As can be seen in Figure 3, these cells were growing exponentially during the first 5.4 hours of cultivation before all the NH₄⁺ in the minimal medium was depleted and the nitrogen-limited growth phase was reached. During the following 3 hours of cultivation the bacterial cells showed a limited linear growth behavior and also a linear progression of glucose consumption. The biomass-specific glucose uptake rate in the nitrogen-limited cultivation phase from hour 5.4 to 8.4 averaged at a value of 0.352 ± 0.016 g_{Glc}/g_{cdw}·h and the biomass-specific growth rate dropped to a value of 0.014 ± 0.002 h⁻¹.

### Example 4:

In this example *Escherichia coli* K-12 MG1655 Δ*relA spoT*[R290E;K292D] *aceE*[G267C] was cultivated under preculture conditions in shaking flasks, as described above, for 29 hours with constant agitation. These bacterial cells were then transferred into the three bioreactors under sterile conditions to start the batch cultivation process. Escherichia coli K-12 MG1655 Δ*relA spoT*[R290E;K292D] *aceE*[G267C] cells were cultivated for a total period of 21.3 hours with a starting concentration of glucose being 28 g/L. In the exponential growth phase the maximal biomass-specific growth rate was 0.290 ± 0.012 h⁻¹ and glucose was consumed at a biomass-specific rate of 1.791 ± 0.059 g_{Glc}/g_{cdw}·h. As can be seen in Figure 4, these cells were growing exponentially during the first 15 hours of cultivation before all the NH₄⁺ in the minimal medium was depleted and the nitrogen-limited growth phase was reached. During the following 6.3 hours of cultivation the bacterial cells showed a limited linear growth behavior and also a linear progression of glucose consumption. The biomass-specific glucose uptake rate in the nitrogen-limited cultivation phase from hour 15 to 21.3 averaged at a value of 0.596 ± 0.023 g_{Glc}/g_{cdw}·h and the biomass-specific growth rate dropped to a negative value of -0.010 ± 0.004 h⁻¹.

### Example 5:

Specific glucose consumption as determined in Examples 1 to 4 above is summarized in the Tables below.

Table 1 shows the comparison of biomass-specific rates in different *Escherichia coli* K-12 MG1655 mutant strains and the wild-type during the nitrogen-limited batch cultivation phase. Values are calculated from at least three parallel fermentations n≥3. For further comparison, the literature value for mₛ^{true} is given in the last row of Table 1. It designates the "true" maintenance coefficient for glucose for non-growing cells at carbon-limitation conditions.

**Table 1**

| | Glucose uptake (N-limited) qs [g/g_{cdw}·h] | | Growth (N-limited) µ [h⁻¹] | |
|---|---|---|---|---|
| Strain | Ø | σ | Ø | σ |
| *E. coli* K-12 MG1655 wild-type | 0.245 | 0.011 | 0.043 | 0.004 |
| *E. coli* K-12 MG1655 *aceE*[G267C] | 0.314 | 0.012 | 0.008 | 0.004 |
| *E. coli* K-12 MG1655 Δ*relA spoT*[R290E;K292D] | 0.352 | 0.016 | 0.014 | 0.002 |
| *E. coli* K-12 MG1655 Δ*relA spoT*[R290E;K292D] *aceE*[G267C] | 0.596 | 0.023 | -0.010 | 0.004 |
| *Escherichia coli* mₛ^{true} | 0.057 | - | 0.000 | 0.000 |

Table 2 shows the comparison of biomass-specific rates in different *Escherichia coli* K-12 MG1655 mutant strains and the wild-type during the initial batch cultivation phase of exponential growth with all nutrients in excess. Values are calculated from at least three parallel fermentations n≥3.

**Table 2**

| | Glucose uptake (Excess) qs[g/g_{cdw}·h] | | Growth (Excess) µ [h⁻¹] | |
|---|---|---|---|---|
| Strain | Ø | σ | Ø | σ |
| *E. coli* K-12 MG1655 wild-type | 1.765 | 0.056 | 0.718 | 0.007 |
| *E. coli* K-12 MG1655 *aceE*[G267C] | 1.512 | 0.022 | 0.201 | 0.004 |
| *E. coli* K-12 MG1655 Δ*relA spoT*[R290E;K292D] | 1.770 | 0.059 | 0.715 | 0.003 |
| *E. coli* K-12 MG1655 Δ*re*/*A spoT*[R290E;K292D] *aceE*[G267C] | 1.791 | 0.059 | 0.290 | 0.012 |

### Discussion:

According to the present invention, increased sugar uptake rates have been achieved in resting cells by specific targeted interventions into *E. coli* metabolism and metabolic regulation.

Concerning metabolic regulation, it is known that the stringent response in *E*. *coli* plays a central role under conditions of limited substrate availability. In this context, the alarmone (p)ppGpp (guanosine penta- or tetraphosphate) is an important signal for the induction and mediation of the regulatory response. Previous studies have shown that an increase in L-lysine production can be achieved by an increase of (p)ppGpp availability following over-expression of (p)ppGpp synthetase I, encoded by the *E. coli* gene *relA.* With respect to *E. coli* metabolism, it has been shown that introduction of an artificial ATPase activity into *E. coli,* leading to a reduction of the available amount of ATP, results in increased glucose uptake.

Thus, an increase in ppGpp synthesis, e.g. by over-expression of *relA,* should be advantageous for the intracellular availability of carbon sources. Further, an artificial reduction of the availability of ATP should result in *E*. *coli* sugar uptake rates.

However, in the present invention, it has been surprisingly found that reduction of ppGpp synthesis by deletion of *relA,* optionally in combination with a reduction of remaining ppGpp synthetase activity of SpoT by introduction of the *spoT*[R290E;K292D] mutation, and optionally in further combination with introduction of the mutation *aceE*[G267C], results in the desired phenotype of increased sugar uptake rates in resting cells which is two- to three-fold higher as compared to wild-type cells.

## Claims

1. A recombinant *Escherichia coli (E. coli)* host cell, wherein said cell comprises at least one of the following mutations in relation to a wild-type cell:
(iv) deletion of the gene *relA* (Δ*relA*);
(v) amino acid substitutions R290E and K292D in the protein guanosine-3',5'-bis pyrophosphate 3'-pyrophosphohydrolase (bifunctional (p)ppGpp synthetase II; SpoT) (*spoT*[R290E;K292D]); and
(vi) amino acid substitution G267C in the protein pyruvate dehydrogenase subunit E1 (AceE) (*aceE*[G267C]).

2. The recombinant host cell according to claim 1, wherein said cell comprises mutation (i).

3. The recombinant host cell according to claim 1 or claim 2, wherein said cell comprises at least two of the mutations (i) to (iii).

4. The recombinant host cell according to claim 3, wherein said cells comprises mutations (i) and (ii).

5. The recombinant host cell according to any one of claims 1 to 4, wherein said cell comprises all three mutations (i) to (iii).

6. The recombinant host cell according to any one of claims 1 to 5, wherein said cell is derived from *E. coli* strain *E. coli* K-12.

7. The recombinant host cell according to claim 6, wherein said cell is derived from *E. coli* strain *E. coli* K-12 substrain MG1655.

8. A method for the biosynthetic production of a product of interest (POI), wherein said POI is produced in a recombinant host cell according to any one of claims 1 to 7.

9. The method according to claim 8, wherein said POI is a protein and said protein is expressed in said recombinant host cell.

10. The method according to claim 8, wherein said POI is an organic molecule and said organic molecule is produced in said recombinant host cell as a metabolite.

11. The method according to claim 10, wherein said organic molecule is selected from the group consisting of pyruvate, lactate, and acetate.

12. The method according to claim 10 or claim 11, wherein the starting material for the production of said metabolite is a sugar.

13. The method according to claim 12, wherein said sugar is selected from the group consisting of glucose, fructose, mannose, xylose, arabinose, and sucrose.

14. The method according to any one of claims 8 to 13, wherein the metabolic activity of the recombinant host cells is reduced by limiting the amount of available nitrogen sources.

15. Use of a recombinant host cell according to any one of claims 1 to 7 for the biosynthetic production of a product of interest (POI).
